Europäisches Patentamt

European Patent Office

Office européen des brevets

(11) Publication number: **0 641 812 A1**

# EUROPEAN PATENT APPLICATION

(21) Application number: **94113045.2**

(22) Date of filing: **22.08.94**

(51) Int. Cl.6: **C08G 18/78**, C08G 18/10,
C08G 18/12, C07C 273/18

(30) Priority: **02.09.93 US 116024**
**02.09.93 US 116141**

(43) Date of publication of application:
**08.03.95 Bulletin 95/10**

(84) Designated Contracting States:
**BE DE ES FR GB IT NL**

(71) Applicant: **MILES INC.**
**One Mellon Center**
**500 Grant St.**
**Pittsburgh, PA 15219-2502 (US)**

(72) Inventor: **Slack, William E.**
**RD 1, Box 100**
**Moundsville, WV 26041 (US)**
Inventor: **Kemp II, Hersel T.**
**214 Riverside Drive**
**New Martinsville, WV 26155 (US)**
Inventor: **Steppan, David D.**
**104 Drury Court**
**Gibsonia, PA 15044 (US)**

(74) Representative: **Braun, Rolf, Dr. et al**
**Bayer AG,**
**Konzernzentrale RP,**
**Patente Konzern**
**D-51368 Leverkusen (DE)**

(54) **Liquid diphenylmethane diisocyanates and processes for their production and use.**

(57) The present invention relates to stable, liquid allophanate-modified diphenylmethane diisocyanates, processes for their production and use, particularly their use in reaction injection molding processes. The stable, liquid MDI of the present invention has an NCO content of 12 to 32.5% and is the reaction product of an aliphatic alcohol and an isomer composition of diphenylmethane diisocyanate comprising from 2 to 60% by weight 2,4'-diphenylmethane diisocyanate and less than 6% by weight of the 2,2'-diphenylmethane diisocyanate, and the rest being 4,4'-diphenylmethane diisocyanate.

EP 0 641 812 A1

## BACKGROUND OF THE INVENTION

Field of the Invention:

The present invention relates to liquid diphenylmethane diisocyanates (MDI) and to processes for their production and use. More specifically, the present invention relates to liquid, alcohol-based, allophanate-modified diphenylmethane diisocyanate prepolymers, methods of making these diisocyanates and methods of using these diisocyanates, particularly in reaction injection molding (RIM) processes.

Brief Description of the Prior Art:

Liquid diphenylmethane diisocyanates and their use in reaction injection molding processes are generally known in the art. U.S. 3,644,457 discloses room temperature stable liquid isocyanates derived from one mole of diphenylmethane diisocyanate and 0.1 to 0.3 mols of poly-1,2-propylene ether glycol.

U.S. 4,055,548 discloses liquid isocyanate prepolymer compositions obtained by reacting polymethylene polyphenylisocyanate containing from about 65 to 85 percent by weight of methylene bis-(phenyl)isocyanate with a polyoxyethylene glycol having molecular weight of from 200 to 600 in an equivalent ratio of 0.0185 - 0.15:1.

U.S. 4,115,429 and 4,118,411 disclose low temperature (as low as -5°C), storage stable liquid diphenylmethane diisocyanates which are produced by reacting diphenylmethane diisocyanates having a specified 2,4-isomer content with propylene glycol or poly-1,2-propylene ether glycol.

U.S. 4,261,852 discloses liquid polyisocyanate compositions comprising (a) the reaction product of 90 to 50% by weight of a reaction product of diphenylmethane diisocyanate and a polyoxypropylene diol or triol having a hydroxyl equivalent weight of from 750 to 3000, said reaction product having an NCO content of from 8 to 26% by weight, and (b) from about 10 to 50% by weight of a diphenylmethane diisocyanate containing from 30 to 65% by weight of diphenylmethane diisocyanate, the remainder being polymethylene polyphenyl polyisocyanate.

U.S. 4,490,300 discloses room temperature stable liquid isocyanates which are derived by reacting diphenylmethane diisocyanate with an aliphatic diol having a pendant aromatic group, e.g., 2-methyl-2-phenyl-1,3-propanediol or phenyl-1-2-ethanediol.

U.S. 4,490,300 discloses room temperature stable liquid isocyanates which are derived by reacting diphenylmethane diisocyanate with monoallylether of trimethylolpropane.

U.S. 4,738,991 discloses organic polyisocyanates characterized by allophanate linkages which are prepared by reacting an organic polyisocyanate including 2,4- and 4,4-methylenediphenyl diisocyanate with poly- or monohydric alcohol in the presence of an organometallic catalyst. The catalyst is then deactivated using a compound such as an inorganic acid, organic acid, organic chloroformate or an organic acid chloride.

U.S. 4,866,103 discloses a polyisocyanate composition for use in producing elastomers in a RIM process, said composition being the reaction product of an alcohol and/or thiol having an average functionality of from about 1.5 to about 4 and an average equivalent weight of at least 500 with at least 2 equivalents per hydroxyl and/or thiol equivalent of an organic polyisocyanate including 4,4- and 2,4-isomers of diphenylmethane diisocyanate. The disclosed reaction is carried out under conditions such that at least about 20% of the initially formed urethane and/or thiourethane groups are converted to allophanate and/or thioallophanate groups.

Another process for the preparation of allophanates which contain isocyanates is disclosed in British Patent 994,890 which relates to the reaction of urethane isocyanates with excess diisocyanate either by heat alone or in the presence of a catalyst such as a metal carboxylate, a metal chelate or a tertiary amine, until the isocyanate content is reduced to that which is obtained theoretically when the complete reaction of the urethane groups is achieved.

U.S. Patent 4,160,080 discloses a process for producing allophanate-containing aliphatically and/or cycloaliphatically bound isocyanate groups. In this disclosed process, compounds containing urethane groups are reacted with polyisocyanates having aliphatic and/or cycloaliphatic isocyanate groups in the presence of a strong acid. The process is generally conducted at a temperature of from 90°C to 140°C for about 4 to 20 hours.

Japanese Patent Application No. 1971-99176 discloses a method of preparing liquid diphenylmethane diisocyanate by reacting diphenylmethane diisocyanate with an aliphatic monovalent alcohol.

Due to the fast reactivity of many of the preferred prepolymers with some of the isocyanate reactive compounds, pertinent among which are amine chain extenders, it has been difficult to get the required high

2

flex modulus while either maintaining or improving other physical properties and being able to fill large molds. Hence, much work has been done to develop MDI based prepolymers which give high flex modulus, at lower amine chain extender levels, and have good RIM processing without sacrificing the other physical properties of the resulting products.

By the present invention there are provided novel liquid isocyanates which are derived from the reaction product of a specified isomer composition of diphenylmethane diisocyanate. These liquid isocyanates represent a new type of MDI based prepolymers which can be RIM processed and also meet the above requirements.

## SUMMARY OF THE INVENTION

In accordance with the foregoing, the present invention, in the first embodiment encompasses a stable, liquid MDI prepolymer comprising an alcohol-based, allophanate-modified MDI having an isocyanate content of about 12 to about 32.5% and characterized in that the allophanate is a reaction product of an aliphatic alcohol and a specified isomer composition of diphenylmethane diisocyanate containing from 2 to 60% by weight 2,4'-diphenylmethane diisocyanate and less than 6% by weight of 2,2'-diphenylmethane diisocyanate, and the rest being 4,4'-diphenylmethane diisocyanate.

In the second embodiment, the invention encompasses a stable, liquid MDI prepolymer having an isocyanate content of about 5 to 30% comprising a reaction product of (a) the allophanate-modified MDI such as described above, and (b) (i) an organic material containing two or more active hydrogen groups which are hydroxyl groups, primary amine groups, secondary amine groups or a combination thereof, (ii) a low molecular weight diol, or (iii) a combination of (i) and (ii).

Further encompassed by the invention are processes for preparing the above-described liquid MDI prepolymers. In the process of the present invention, the allophanate-modified MDI can be prepared by first pre-reacting the specified diphenylmethane diisocyanate with an aliphatic alcohol to form a urethane which is subsequently converted to an allophanate. Alternately, the aliphatic alcohol and the diphenylmethane diisocyanate and the appropriate catalyst can be reacted to form the allophanate directly.

The resultant allophanate-modified MDI having an isocyanate content of about 12 to 32.5% can be reacted with a high molecular weight organic material containing two or more active hydrogen atoms which can be hydroxyl, primary amine, secondary amine or a combination thereof, having a molecular weight of from 400 to 6000, and/or with a low molecular weight organic material containing two or more hydroxyl groups which are typically diols having a molecular weight of from 60 to 200. The resultant product is a stable, liquid MDI prepolymer with an isocyanate group content of from 5 to 30% by weight.

In a preferred embodiment of the present invention, the allophanate can be prepared by reacting the specified diphenylmethane diisocyanate with an aliphatic alcohol at about 20°C to about 115°C. The resultant urethane is converted to an allophanate at 60-110°C, using zinc acetylacetonate as a catalyst, and benzoyl chloride as a catalyst stopper in a weight ratio of benzoyl chloride to zinc acetylacetonate of 2:1.

It is a distinct feature of the isocyanates of the present invention that they are stable and liquid at 25°C. By the term "stable" is meant that the prepolymer has up to 1% absolute change in the NCO content and up to 10% change in the viscosity when stored at 25°C for 3 months. The term "liquid" means that the prepolymer does not precipitate solids when stored at 25°C for 3 months.

The present invention also encompasses a process for injecting a reaction mixture into a closed mold via a reaction injection molding (RIM) machine with the ratio of the components being such that the isocyanate index is about 80 to 120, said reaction mixture comprising: (i) an isocyanate reactive material, (ii) a chain extender, and (iii) a storage stable liquid MDI prepolymer comprising an alcohol-based, allophanate-modified MDI having an isocyanate content of about 12 to 32.5% and characterized in that the allophanate is a reaction product of an aliphatic alcohol and specified isomer composition of diphenylmethane diisocyanate containing from 2 to 60% by weight 2,4'-diphenylmethane diisocyanate and less than 6% by weight of the 2,2'-diphenylmethane diisocyanate, and the rest being 4,4'-diphenylmethane diisocyanate.

In another embodiment, the invention encompasses the use of a stable, liquid MDI prepolymer having an isocyanate content of about 5 to 30% comprising a reaction product of (A) the allophanate-modified MDI such as described above, and (B) (i) an organic material containing two or more active hydrogen groups which are hydroxyl groups, primary amine groups, secondary amine groups or a combination thereof (ii) a low molecular weight diol, or (iii) a combination of (i) and (ii).

The molded products prepared by the above processes are also encompassed by the invention.

The liquid isocyanate prepolymers of the invention have been found to be particularly useful in automotive reaction injection molding (RIM)'s and rigid foam applications.

3

DETAILED DESCRIPTION OF THE INVENTION

In accordance with the invention, the stable, liquid MDI prepolymers of the present invention may be used in a RIM process without compromising the performance properties of the resulting molded products. Surprisingly, the use of these prepolymers results in molded products having increased flex modulus.

The stable, liquid MDI prepolymer of the invention contains an aliphatic alcohol-based, allophanate-modified MDI characterized in that it is a reaction product of an aliphatic alcohol and a specified isomer composition of MDI which is effective to produce an MDI prepolymer that is stable and liquid and has desirable performance properties.

In one embodiment of the present invention, a novel, stable, liquid MDI prepolymer containing an allophanate-modified MDI having an NCO content of 12 to 32.5% is obtained by reaction of an aliphatic alcohol and a specified isomer composition of MDI. The isomer composition of MDI is from about 6 to 60% (preferably about 2 to about 25%) by weight 2,4'-diphenylmethane diisocyanate and less than 6% (preferably about 0 to 0.7%) by weight of the 2-,2'-diphenylmethane diisocyanate, and the rest being 4,4'-diphenylmethane diisocyanate. The allophanate-modified MDI has an NCO content of about 12 to 32.5% (preferably 20 to 31% by weight) is particularly useful in RIM processes.

In another embodiment of the present invention, the allophanate modified MDI has an NCO content of from about 5 to about 30% (preferably from about 10 to about 25%) by weight. This MDI is also useful in the production of molded parts by RIM processes.

Aliphatic alcohols useful herein are those that can react with the diphenylmethane diisocyanate to form allophanates in accordance with the invention. Useful aliphatic alcohols can contain about 1 to 36 and preferably 4 to 16 carbon atoms. Illustrative examples of suitable aliphatic alcohols include: aliphatic alcohols, cycloaliphatic alcohols, aliphatic alcohols containing aromatic groups, aliphatic alcohols containing groups that do not react with isocyanates (e.g., ether groups and halogens such as chlorine and bromine). Specific but non-limiting examples of the aliphatic alcohols include 1-butanol, cetylalcohol, cyclohexanol, 2-methoxyethanol, and 2-bromoethanol.

The allophanate-modified MDI of the present invention may be prepared by (1) reacting an aliphatic alcohol with the MDI having the above-specified isomer composition at a temperature of 20 to 115°C to form a urethane and (2) adding a catalyst to the urethane formed in (1) at 60 to 120°C to form the allophanate.

Alternately, the catalyst and the alcohol can be added together to the MDI at 40-60°C, and the resulting mixture can be heated to about 60 to 120°C and typically at 90°C to form the allophanate.

The catalyst in the resultant product is typically neutralized before the reaction with organic materials containing hydroxyl groups or amine groups as described hereinafter. As such the catalysts useful herein are those that can be neutralized or otherwise stopped from adversely affecting subsequent reactions.

Illustratively, a catalyst such as zinc acetyl-acetonate can be employed, and a catalyst stopper such as an acidic material (e.g., anhydrous hydrochloric acid, sulfuric acid, bis(2-ethylhexyl)hydrogen phosphate, benzoyl chloride, Lewis acids and the like) in the ratio of 2 equivalents of the acid to each mole of the zinc acetylacetonate.

Other allophanate catalysts such as zinc 2-ethyl-hexanoate, cobalt 2-ethylhexanoate, cobalt naphthenate, lead linoresinate can be employed. Solvents which are typically inert to the isocyanate, for example toluene, tetrahydrofuran or o-dichloro-benzene can be employed.

In another embodiment of the invention, (A) a stable, liquid prepolymer containing the reaction product of the allophanate-modified MDI, having an isocyanate group content of from 12.0 to 32.5% is reacted with (B) (i) an organic material containing two or more and preferably 2 to 3 active hydrogen groups which can be hydroxyl groups, primary amine groups, secondary amine groups or a combination thereof having a molecular weight of from 400 to 6000, and preferably 1000 to 5000 or (ii) with an organic material preferably containing two or more and preferably two hydroxyl groups, having a molecular weight of from 60 to 200 and preferably 76 to 90 or a combination of (i) and (ii). The resultant isocyanate prepolymer has an isocyanate group content of about 5 to 30% by weight. The urethane, urea, or biuret reaction is carried out in a manner which is well known in polyurethane chemistry by, say, heating the reactants to a temperature of from about 40 to 150°C and preferably 50 to 100°C to form the urethane or urea and at 100 to 150°C and preferably 110 to 120°C to form the biuret.

The useful organic materials containing two or more hydroxyl groups having a molecular weight of 400 to 6000 can be a polyol selected from the group consisting of polyester polyols, polyether polyols, polyhydroxy polycarbonates, polyhydroxy polyacetals, polyhydroxy polyacrylates, polyhydroxy polyester amides and polyhydroxy polythioethers. The polyester polyols, polyether polyols and polyhydroxy polycarbonates are preferred.

Suitable polyester polyols include reaction products of polyhydric, preferably dihydric alcohols to which trihydric alcohols may be added and polybasic, preferably dibasic carboxylic acids. Instead of these polycarboxylic acids, the corresponding carboxylic acid anhydrides or polycarboxylic acid esters of lower alcohols or mixtures thereof may be used for preparing the polyesters. The polycarboxylic acids may be aliphatic, cycloaliphatic, aromatic and/or heterocyclic and they may be substituted (e.g., by halogen atoms) and/or unsaturated. The following are mentioned as examples: succinic acid; adipic acid; suberic acid; azelaic acid; sebacic acid; phthalic acid; isophthalic acid; trimellitic acid; phthalic acid anhydride; tetrahydrophthalic acid anhydride; hexahydrophthalic acid anhydride; tetrachlorophthalic acid anhydride, endomethylene tetrahydrophthalic acid anhydride; glutaric acid anhydride; maleic acid; maleic acid anhydride; fumaric acid; dimeric and trimeric fatty acids such as oleic acid, which may be mixed with monomeric fatty acids; dimethyl terephthalates and bis-glycol terephthalate. Suitable polyhydric alcohols include, e.g. ethylene glycol; propylene glycol-(1,2) polyhydric alcohols include, e.g. ethylene glycol; propylene glycol-(1,2) and -(1,3); butylene glycol-(1,4) and -(1,3); hexanediol-(1,6); octanediol-(1,8); neopentyl glycol; cyclohexanedimethanol; (1,4-bis-hydroxymethylcyclohexane); 2-methyl-1,3-propanediol; 2,2,4-trimethyl-1,3-pentanediol; triethylene glycol; tetraethylene glycol; polyethylene glycol; dipropylene glycol; polypropylene glycol; dibutylene glycol and polybutylene glycol, glycerine and trimethlyolpropane. The polyesters may also contain a portion of carboxyl end groups. Polyesters of lactones (e.g., $\epsilon$-caprolactone) or hydroxycarboxylic acids (e.g., $\omega$-hydroxycaproic acid) may also be used.

Polycarbonates containing hydroxyl groups include those known per se such as the products obtained from the reaction of diols such as propanediol-(1,3), butanediol-(1,4) and/or hexanediol-(1,6), diethylene glycol, triethylene glycol or tetraethylene glycol with phosgene, diarylcarbonates such as diphenylcarbonate or with cyclic carbonates such as ethylene or propylene carbonate. Also suitable are polyester carbonates obtained from the above-mentioned polyesters or polylactones with phosgene, diaryl carbonates or cyclic carbonates.

Suitable polyether polyols are obtained in known manner by the reaction of starting compounds which contain reactive hydrogen atoms with alkylene oxides such as ethylene oxide, propylene oxide, butylene oxide, styrene oxide, tetrahydrofuran, epichlorohydrin or mixtures of these alkylene oxides. It is preferred that the polyethers do not contain more than about 20% by weight of ethylene oxide units. Suitable starting compounds containing reactive hydrogen atoms include the polyhydric alcohols set forth for preparing the polyester polyols and, in addition, water, methanol, ethanol, 1,2,6-hexane triol, 1,2,4-butane triol, trimethylol ethane, pentaerythritol, mannitol, sorbitol, methyl glycoside, sucrose, phenol, isononyl phenol, resorcinol, hydroquinone, 1,1,1- or 1,1,2-tris-(hydroxylphenyl)-ethane.

Polyethers modified by vinyl polymers are also suitable for the process according to the invention. Products of this kind may be obtained by polymerizing, e.g. styrene and acrylonitrile in the presence of polyethers (U.S. Patent Nos. 3,383,351; 3,304,273; 3,523,095; 3,110,695 and German Patent No. 1,152,536).

Among the polythioethers which should be particularly mentioned are the condensation products obtained from thiodiglycol on its own and/or with other glycols, dicarboxylic acids, formaldehyde, aminocarboxylic acids or amino alcohols. The products obtained are either polythio-mixed ethers, polythioether esters or polythioether ester amides, depending on the co-components.

Amine-terminated polyether useful herein can be prepared by reacting a primary amine or ammonia with a polyether containing a terminal leaving group such as halides, or mesylates as disclosed in commonly assigned U.S. Patent Application Serial Number 07/957,929, filed on October 7, 1992, or as disclosed in U.S. Patents 3,666,726; 3,691,112; and 5,066,824.

Suitable polyacetals include the compounds which can be prepared from aldehydes, e.g. formaldehyde, and glycols such as diethylene glycol, triethylene glycol, ethoxylated 4,4'-di-hydroxy-diphenyl-dimethylmethane, and hexanediol-(1,6). Polyacetals suitable for the purpose of the invention may also be prepared by the polymerization of cyclic acetals.

Suitable polyhydroxy polyester amides and polyamines include the predominantly linear condensates obtained from polybasic saturated and unsaturated carboxylic acids or their anhydrides and polyvalent saturated or unsaturated aminoalcohols, diamines, polyamines and mixtures thereof.

Suitable monomers for producing hydroxy-functional polyacrylates include acrylic acid, methacrylic acid, crotonic acid, maleic anhydride, 2-hydroxyethyl acrylate, 2-hydroxyethyl methacrylate, 2-hydroxypropyl acrylate, 2-hydroxypropyl methacrylate, 3-hydroxypropyl acrylate, 3-hydroxypropyl methacrylate, glycidyl acrylate, glycidyl methacrylate, 2-isocyanatoethyl acrylate and 2-isocyanatoethyl methacrylate.

The low molecular weight material containing two or more hydroxyl groups having an average molecular weight of 60 to 200 may be used in combination with or instead of the high molecular weight material containing two or more hydroxyl groups. They include the polyhydric alcohols which have previously been described for the preparation of the polyester polyols and polyether polyols. Dihydric alcohols are preferred.

The weight ratio of the low molecular weight to the high molecular weight material containing two or more hydroxyl groups can be from 0.001 to 2 and preferably 0.01 to 0.40 .

In addition to the above-mentioned components which are preferably difunctional, monofunctional and even small portions of trifunctional and higher functional components generally known in polyurethane chemistry, such as trimethylolpropane, may be used in special cases in which slight branching of the resultant product is desired.

The hydroxy functional materials may be reacted with the allophanate at a temperature within the range of from about 40 to about 150°C, preferably about 50 to 100°C, over a period of 0.1 to 2 hours. Catalysts and solvents can be employed to aid the reaction. Examples of useful catalysts include: di-n-butyltin dichloride, di-n-butyltin-diacetate, di-n-butyltin dilaurate, triethylenediamine, and bismuth nitrate. Examples of useful solvents include: dioxane, chlorobenzene, toluene, and tetrahydrofuran.

The liquid isocyanate prepolymers of the present invention are useful in the preparation of polyurethanes, particularly in the preparation of polyurethanes by reaction injection molding techniques.

The isocyanate prepolymers of the present invention may be molded by a RIM process which is carried out by any of the known techniques in which a reaction mixture is injected into a mold via a RIM machine. Two streams are generally employed in this molding technique. In the present invention, the isocyanate reactive component (i), the chain extender component (ii), and the stable, liquid MDI prepolymer component (iii), additives and auxiliaries can be injected into the mold as follows. Typically, the isocyanate prepolymer component is the first reactant. The isocyanate reactive component and the chain extender component, commonly referred to as the "polyol blend", constitute the second reactant. If any auxiliary agents or additives are used, they are generally mixed with the isocyanate reactive component. It is possible in principle to use mixheads in which three or four separate components may be simultaneously introduced so that no preliminary mixing of the individual components is required. The quantity of reaction mixture introduced into the mold is generally calculated to produce molded articles having densities of from 0.8 to 1.4 g/cm$^3$, preferably from 0.9 to 1.2 g+/cm$^3$. When mineral fillers are used, however, the molded articles may have densities above 1.2 g/cm$^3$. The articles may be removed from the mold after they have been left in there from 5 to 90 seconds, preferably from 20 to 60 seconds.

The reactant mixture is generally introduced into the mold at a starting temperature of from 10 to 60°C, preferably from 20 to 50°C. The temperature of the mold itself is generally from 40 to 100°C, preferably from 50 to 70°C.

As the isocyanate reactive materials there can be employed organic materials containing active hydrogen groups such as hydroxyl groups, primary amine groups, secondary amine groups or a combination thereof having a molecular weight of about 400 to 12000 and preferably about 800 to 3500 and a functionality of 2 to 6 and preferably 2 to 4. The isocyanate reactive materials can be used in quantities from 50 to 95 and preferably 60 to 90 weight % of the "polyol blend". These organic materials can be prepared in essentially the same manner as described above.

As chain extenders there can be employed organic material containing active hydrogen groups such as hydroxyl, primary or secondary amine groups or a combination thereof having molecular weight of about 62 to 400 and preferably 100 to 300. The chain extenders can be used in quantities of from 5 to 50 wt. %, preferably from 10 to 40 wt. % (based on the weight of the "polyol blend").

The diamines useful as chain extenders in the process of the present invention generally have molecular weights of from 108 to 400 and preferably contain exclusively aromatically bound primary or secondary (preferably primary) amino groups. Examples of such diamines are: 1,4-diaminobenzene, 2,4-diaminotoluene, 2,4- and/or 4,4'-diaminodiphenyl methane, 3,3'-4,4'-diaminodiphenyl methane, 4,4'-diaminodiphenylpropane-(2,2),mixtures of such diamines and the like.

The preferred diamines have alkyl substituents in at least one position which is ortho to the amino groups. The most preferred diamines are those in which at least one alkyl substituent is present in the position ortho to the first amino group and two alkyl substituents are located in the position ortho to the second amino group, each alkyl substituent having 1 to 4 carbon atoms. It is particularly preferred to use such compounds in which an ethyl, n-propyl, isopropyl, t-butyl and/or methylthio substituent is present in at least one position ortho to the amino groups and possibly methyl substituents in other positions ortho to the amino groups.

Specific examples of preferred amines include 2,4-diaminomesitylene, 1,3,5-triethyl-2,4-diaminobenzene, 1,3,5-triisopropyl-2,4-diaminobenzene,1-methyl-3,5-diethyl-2,4-diaminobenzene, 1-methyl-3,5-diethyl-diaminobenzene, 4,6-dimethyl-2-ethyl-1,3-diaminobenzene, 3,5,3',5'-tetra-ethyl-4,4'-diaminodiphenyl methane, 3,5,3',5'-tetraisopropyl-4,4'-diaminodiphenyl methane, 3,5-diethyl-3,5-diisopropyl-4,4-diaminodiphenyl methane, t-butyl toluenediamine and bis-thiomethyl toluene diamine. Also useful are adducts of these amines with epoxy resins. It is also within the scope of this invention to use aliphatic amine chain extender

6

materials as described in U.S. Patents 4,246,363, 4,269,945, 4,495,081 and 4,530,941, although the aliphatic amines are not preferred.

The above mentioned diamines may, of course, also be used as mixtures. It is particularly preferred to use 1-methyl-3,5-diethyl-2,4-diamino-benzene or a mixture of this compound with 1-methyl-3,5-diethyl-2,6-diaminobenzene.

The diamine chain extending agent in the process of the present invention is preferably used in quantities of from 5 to 50 wt. %, most preferably from 10 to 40 wt. % (based on the weight of the polyol blend).

Also useful herein are diols such as described above, e.g., ethylene glycol, 1,2- and 1,3-propane diol, 1,3- and 1,4- and 2,3-butane diol, 1,6-hexane diol, diethylene glycol, triethylene glycol, tetraethylene glycol, dipropylene glycol, tripropylene glycol, glycerol, trimethylol propane, and pentaerythritol.

Known mold release agents may be used to produce molded articles which have excellent mold release characteristics. Such internal mold release agents are among the auxiliary agents which may advantageously be used in the process of the present invention. In principle, any mold release agent known in the art may be used in the present invention but internal mold release agents such as those described, for example, in German Offenlegungsschrift No. 1,953,637 (U.S. 3,726,952), German Offenlegungsschrift 2,121,670 (British Patent 1,365,215), German Offenlegungsschrift 2,431,968 (U.S. 4,098,731), German Offenlegungsschrift 2,404,310 (U.S. 4,058,492) and U.S. Patents 4,519,965 and 4,581,386 are preferred. Preferred mold release agents include the salts (containing at least 25 aliphatic carbon atoms) of fatty acids having at least 12 aliphatic carbon atoms and primary mono-, di- or polyamines containing two or more carbon atoms or amines containing amide or ester groups and having at least one primary, secondary or tertiary amino group; esters of mono- and/or polyfunctional carboxylic acids and polyfunctional alcohols containing saturated and/or unsaturated COOH and/or OH groups and having hydroxyl or acid numbers of at least five, ester type reaction products of ricinoleic acid and long chained fatty acids; salts of carboxylic acids and tertiary amines; and natural and/or synthetic oils, fats or waxes. Also preferred are the zinc salts described in U.S. 4,519,965 and 4,581,386.

The oleic acid or tall oil fatty acid salts of the amine containing amide groups which has been obtained by the reaction of N-dimethylaminopropylamine with oleic acid or tall oil fatty acid is particularly preferred.

Apart from the above-described preferred mold release agents, other mold release agents known in the art may in principle be used either alone or in a mixture with the preferred mold release agents. These additional mold release agents include, for example, the reaction products of fatty acid esters with polyisocyanates (according to German Offenlegungsschrift 2,319,648); the reaction products of polysiloxanes containing reactive hydrogen atoms with mono- and/or polyisocyanates (according to German Offenlegungsschrift 2,356,692 (U.S. 4,033,912)); esters of mono- and/or polycarboxylic acids and polysiloxanes containing hydroxyl groups (according to German Offenlegungsschrift 2,363,452 (U.S. 4,024,090)); and salts of polysiloxanes containing amino groups and fatty acids (according to German Offenlegungsschrift 2,417,273 or German Offenlegungsschrift 2,431,968 (U.S. 4,098,731)).

If an internal mold release agent is used, it is generally used in an amount which totals from 0.1 to 25 wt. %, preferably 1 to 10 wt. % of the whole reaction mixture.

No catalyst is required for the reaction between isocyanate groups and isocyanate reactive groups of the components b) and c). However, catalysts known and commonly used in the production of polyurethane foams and microcellular elastomers are included in the group of auxiliary agents and additives appropriate to the present invention.

Suitable catalysts include tertiary amines such as triethylamine, tributylamine, N-methyl-morpholine, N-ethylmorpholine, N-cocomorpholine, N,N,N',N'-tetramethylethylene diamine, 1,4-diazabicyclo-(2,2,2)-octane, N-methyl-N'-dimethylaminoethyl piperazine, N,N-dimethylbenzylamine, bis-(N,N-diethylamino)-adipate, N,N-diethyl benzylamine, pentamethyl diethylene triamine, N,N-dimethylcyclohexylamine, N,N,N',N'-tetramethyl-1,3-butanediamine, 1,2-dimethylimidazole and 2-methylimidazole.

Organometallic catalysts may also be used in the practice of the present invention. Particularly useful organometallic catalysts include organic tin catalysts such as tin(II) salts of carboxylic acids (e.g., tin-(II)-acetate, tin-(II)-laurate) and the dialkyl tin salts of carboxylic acids (e.g., dibutyl-tin-diacetate, dibutyl-tin-dilaurate, dibutyl-tin-maleate or dioctyl-tin-diacetate) alone or in combination with tertiary amines. Other suitable catalysts and details concerning the action of these catalysts are given in Kunststoff Handbuch, Volume VII, published by Vieweg and Hochtlen, Carl Hanser Verlag, Munich 1966, e.g., on pages 96 to 102.

If a catalyst is used, quantities of about 0.001 to 10 wt. %, preferably 0.05 to 1 wt. % (based on the polyol blend) are appropriate.

The products of the process of the present invention are preferably molded elastomeric articles. Blowing agents may be used to produce molded articles having a compact surface and a cellular interior.

The blowing agents used may be water and/or readily volatile organic substances and/or dissolved inert gases.

Examples of suitable organic blowing agents include acetone; ethylacetate; methanol; ethanol; halogen-substituted alkanes such as methylene chloride, chloroform, ethylidene chloride, vinylidene chloride, monofluorotrichloromethane, chlorodifluoromethane and dichlorofluoromethane; and butane, hexane, heptane or diethyl ether.

Nitrogen, air and carbon dioxide are examples of suitable inert gases.

The effect of a blowing agent may also be obtained by the addition of compounds which decompose at temperatures above room temperature to release gases, for example nitrogen. Azo compounds such as azoisobutyric acid nitrile are examples of such compounds. Other examples of blowing agents and details concerning the use of blowing agents may be found in Kunststoff Handbuch, Volume VII, published by Vieweg and Hochtlen, Carl Hanser Verlag, Munich 1966, e.g., on pages 108 and 109, 453 to 455 and 507 to 510.

Surface active additives (emulsifiers and foam stabilizers) may also be used as reaction mixture components. Suitable emulsifiers include the sodium salts of ricinoleic sulfonates or of fatty acids or salts of fatty acids and amines (such as oleic acid diethylamine or stearic acid diethanolamine). Alkali metal or ammonium salts of sulfonic acids (e.g., of dodecyl benzene sulfonic acid or of dinaphthyl methane disulfonic acid) or of fatty acids such as ricinoleic acid or polymeric fatty acids may also be used as surface active additives.

If foam stabilizers are used, it is preferred that they be water soluble polyether siloxanes. These compounds are generally a copolymer of ethylene oxide and propylene oxide linked to a polydimethyl siloxane group. Foam stabilizers of this type are described in U.S. 2,764,565.

Other auxiliary agents and additives which may optionally be used in the process of the present invention include known cell regulators (such as paraffins or fatty alcohols or dimethyl polysiloxanes), known pigments, dyes and flame retarding agents (e.g., tris-chloroethyl phosphate and polyphosphate), stabilizers against aging and weathering, plasticizers, fungistatic and bacteriostatic substances, and fillers (such as barium sulfate, glass fibers, kieselguhr or whiting).

Other examples of suitable surface active additives and foam stabilizers, flame retardants, plasticizers, dyes, fillers and fungistatic and bacteriostatic substances and details concerning the use of mode of action of these additives may be found in Kunststoff Handbuch, Volume VII, published by Vieweg and Hochtlen, Carl Hanser Verlag, Munich 1966, e.g., on pages 103 to 113.

When carrying out the process of the present invention, the quantity of polyisocyanates (component (iii)) should preferably be such that the isocyanate index is from 70 to 130, most preferably 90 to 110 in the reaction mixture.

The process is advantaged by the use of liquid prepolymers to efficiently produce RIM products with good processing and performance properties. It is a distinct feature of the invention that the flex modulus of the RIM products can be increased (to about 60,000 to 90,000 psi) without modifying the chain extender composition or content. It is also a distinct feature of the invention that relatively low (below the conventional levels of 33 parts by weight) but effective levels of chain extenders can be employed to impart substantial improvement in flex modulus. As would be realized, in using low levels of chain extender, one can avoid the expense, and reactivity problems associated with higher levels of chain extenders.

The molded articles obtainable by the process of the present invention are particularly suitable for the manufacture of flexible automobile bumpers or car body parts.

The invention is further illustrated but is not intended to be limited by the following examples in which all parts and percentages are by weight unless otherwise specified.

EXAMPLES

The first step in the preparation of these prepolymers is the preparation of the allophanate. The allophanate can be prepared in a two-step process.

First, the urethane is prepared by reacting the aliphatic alcohol and the diphenylmethane diisocyanate (MDI) at 60°C, followed by the addition of catalyst to the resulting urethane in order to form the allophanate. Alternately, the catalyst and the alcohol can be added together to the MDI at 40-60°C, and the resulting mixture is heated at between 60 and about 90°C. The catalyst in the resultant product is neutralized before the addition of other hydroxy or amino containing compounds. Illustratively, zinc acetylacetonate catalyst, can be neutralized using acidic materials such as anhydrous hydrochloric acid, sulfuric acid, bis(2-ethylhexyl)hydrogen phosphate, benzoyl chloride, Lewis acids and the like in the ratio of 2 equivalents of the acid to each mole of the zinc acetylacetonate.

8

The combination of diol and polyether polyol or amine terminated polyether can be added to the allophanate in any order.

The following materials were used in the preparation of the prepolymers:

Polyol A: a propylene glycol/propylene oxide adduct having a 2000 molecular weight and a hydroxyl value of 56.

Polyol B: a glycerine/propylene oxide/ethylene oxide adduct with the ethylene oxide being present as a 17% by weight termination, having a 4800 molecular weight and a hydroxyl value of 35.

Polyol C: a glycerine/propylene oxide adduct having a 3000 molecular weight and a hydroxyl value of 56.

Polyol D: propylene glycol/propylene oxide adduct having a 1000 molecular weight and a hydroxyl value of 112.

Polyol E: a glycerine/propylene oxide/ethylene oxide adduct with the ethylene oxide being present as a 13% by weight termination, having a 6000 molecular weight and a hydroxyl value of 28.

Amine Terminated Polyether Preparation (ATPE)

Polyol D (2.6 eq.), triethylamine (2.91 mole), and 300 ml methylene chloride were added to a 3 liter, 3-neck flask fitted with a stirrer and reflux condenser. Methane sulfonyl chloride (2.91 mole) was added dropwise, keeping the solution temperature at 25°C with an ice water bath. The reaction solution was stirred at room temperature for 0.5 hours, then neutralized with sodium hydroxide (2.91 mole). Triethylamine, solvent and water were vacuum stripped and the product filtered to give the mesylate as a clear, colorless liquid.

This mesylate (1.9 eq.) was added to a 3 liter, 3-necked flask fitted with a stirrer and reflux condenser. 2-Ethyl-hexylamine (5.7 mole) was added and the solution was heated at 150°C for 9 hours. The solution was cooled and neutralized with sodium hydroxide (1.9 mole). Excess amine and water were vacuum stripped and the product filtered to give a clear, light yellow liquid with a viscosity of 105 mPa•s (at 25°C) and an amine number of 83.0. This ATPE was used in Examples 45 and 46.

PG: propylene glycol

1,3 XB: 1,3-butanediol

MDI-X: Diphenylmethane diisocyanate which contains less than 6% by weight 2,2'-isomer of diphenylmethane diisocyanate and in which X represents the percent by weight 2,4'-isomer of diphenylmethane diisocyanate with the remainder being the 4,4'- and 2,2'-isomers.

Isocyanate A: 100 parts (PBW) of MDI-2 and 1 part of 1-butanol were charged to a stirred reactor and heated to 60°C. 0.01 part of zinc acetylacetonate was added and the stirred reaction mixture was heated to 90°C. After one hour at 90°C the NCO content was 32.1%. The reaction mixture was cooled to 60°C and 0.025 part of benzoyl chloride was added. The reaction mixture was cooled to 40°C and stored at 40°C until used.

Isocyanates B through I: were prepared in essentially the same manner as described for Isocyanate A. The materials used and the % NCO obtained are set forth in Table 1. The same catalyst and stopper used for Isocyanate A were used for Isocyanates B through I, in the same amounts.

9

## Table 1

| Isocyanate | MDI-X | PBW MDI-X | Alcohol Used | PBW Alcohol | NCO Content % by weight |
|---|---|---|---|---|---|
| B | 2 | 100 | 1-butanol | 2.0 | 30.7 |
| C | 2 | 100 | 1-butanol | 3.0 | 29.3 |
| D | 2 | 100 | 1-butanol | 4.7 | 27.0 |
| E | 10 | 100 | 1-butanol | 1.0 | 32.1 |
| F | 10 | 100 | 1-butanol | 2.0 | 30.7 |
| G | 20 | 100 | 1-butanol | 2.0 | 30.7 |
| H | 2 | 100 | methanol | 1.2 | 30.0 |
| I | 15 | 100 | 1-butanol | 4 | 28.0 |

Example 1

63.9 parts of Isocyanate A were charged to a reactor and heated to 60°C. 34.2 parts of Polyol B and 1.9 parts of PG were added to the stirred isocyanate at such a rate that the temperature was maintained at 60°C ± 5°. The reaction mixture was held at 60°C for about 2 hours and then cooled to 25°C. The resultant product, which had an isocyanate group content of 17.5%, was a clear liquid and storage stable at 25°C.

Examples 2 through 44

Examples 2 through 44 used the process of Example 1. All the products were clear liquids and storage stable at 25°C. The materials used and the percent NCO results obtained were as set forth in Table 2.

Example 45

150 parts of Isocyanate I were charged to a reactor and heated to 40°C. 0.033 part of benzoyl chloride was added. 31.8 parts of the ATPE was added over a ten minute period with good stirring. The reaction mixture was held at 50°C for 12 minutes then cooled to room temperature. The resultant urea modified product, which has an isocyanate group content of 22.0%, and a viscosity of 265 mPa•s at 25°C was a clear liquid and storage stable at 25°C.

Example 46

Example 45 was repeated except after being heated at 50°C for 12 minutes, the material was heated up to 120°C and held for 90 minutes followed by cooling to 25°C. The resultant biuret modified product, which has an isocyanate group content of 20.6% and a viscosity of 780 mPa•s at 25°C, was a clear liquid and storage stable at 25°C.

## Table 2

| Ex. | Isocyanate Used | PBW Isocyanate | Polyol Used | PBW Polyol | Diol Used | PBW Diol | NCO Content % by weight |
|-----|-----------------|----------------|-------------|------------|-----------|----------|-------------------------|
| 2 | A | 64.8 | A | 32.6 | 1,3 XB | 2.6 | 16.9 |
| 3 | A | 68.3 | A | 31.0 | 1,3 XB | 0.7 | 19.9 |
| 4 | B | 60.8 | C | 39.2 | - | - | 17.0 |
| 5 | B | 70.1 | D | 29.9 | - | - | 19.2 |
| 6 | B | 58.7 | B | 39.5 | PG | 1.8 | 15.6 |
| 7 | B | 62.3 | B | 36.5 | 1,3 XB | 1.2 | 17.0 |
| 8 | B | 73.8 | A | 24.7 | PG | 1.5 | 19.8 |
| 9 | C | 83.3 | D | 16.7 | - | - | 22.9 |

EP 0 641 812 A1

## Table 2- Continued

| Ex. | Isocyanate Used | PBW Isocyanate | Polyol Used | PBW Polyol | Diol Used | PBW Diol | NCO Content % by weight |
|-----|-----------------|----------------|-------------|------------|-----------|----------|--------------------------|
| 10 | C | 60.8 | E | 39.2 | - | - | 17.1 |
| 11 | C | 81.2 | A | 18.8 | - | - | 22.4 |
| 12 | C | 80.3 | B | 19.7 | - | - | 23.1 |
| 13 | C | 77.2 | A | 21.3 | PG | 1.5 | 20.3 |
| 14 | C | 70.9 | A | 26.3 | PG | 2.8 | 16.5 |
| 15 | D | 94.3 | - | - | 1,3 XB | 5.7 | 20.3 |
| 16 | D | 79.2 | A | 18.4 | PG | 2.4 | 18.0 |
| 17 | D | 79.3 | B | 17.5 | 1,3 XB | 3.2 | 17.8 |

EP 0 641 812 A1

## Table 2- Continued

| Ex. | Isocyanate Used | PBW Isocyanate | Polyol Used | PBW Polyol | Diol Used | PBW Diol | NCO Content % by weight |
|-----|-----------------|----------------|-------------|------------|-----------|----------|-------------------------|
| 18 | D | 76.4 | B | 23.6 | - | - | 20.0 |
| 19 | D | 77.6 | C | 22.4 | - | - | 20.1 |
| 20 | D | 75.9 | E | 24.1 | - | - | 19.9 |
| 21 | E | 66.7 | C | 33.3 | - | - | 20.0 |
| 22 | E | 64.6 | E | 35.4 | - | - | 20.0 |
| 23 | E | 67.0 | B | 29.7 | PG | 3.3 | 17.0 |
| 24 | E | 79.8 | B | 14.6 | 1,3 XB | 5.6 | 20.1 |
| 25 | E | 68.5 | A | 28.1 | PG | 3.4 | 17.0 |

## Table 2- Continued

| Ex. | Isocyanate Used | PBW Isocyanate | Polyol Used | PBW Polyol | Diol Used | PBW Diol | NCO Content % by weight |
|-----|------|------|------|------|------|------|------|
| 26 | F | 69.3 | C | 30.7 | - | - | 20.1 |
| 27 | F | 67.4 | E | 32.6 | - | - | 20.0 |
| 28 | F | 98.0 | - | - | 1,3 XB | 2.0 | 28.3 |
| 29 | F | 77.2 | A | 19.7 | 1,3 XB | 3.1 | 19.9 |
| 30 | F | 76.2 | B | 20.7 | 1,3 XB | 3.1 | 20.1 |
| 31 | G | 79.5 | A | 16.5 | 1,3 XB | 4.0 | 20.0 |
| 32 | G | 81.1 | B | 14.0 | 1,3 XB | 4.9 | 19.8 |
| 33 | G | 78.0 | B | 18.9 | PG | 3.1 | 20.0 |

Table 2- Continued

| Ex. | Isocyanate Used | PBW Isocyanate | Polyol Used | PBW Polyol | Diol Used | PBW Diol | NCO Content % by weight |
|---|---|---|---|---|---|---|---|
| 34 | G | 96.2 | - | - | 1,3 XB | 3.8 | 25.8 |
| 35 | G | 69.3 | A | 30.7 | - | - | 20.3 |
| 36 | G | 69.3 | C | 30.7 | - | - | 20.3 |
| 37 | G | 67.4 | E | 32.6 | - | - | 20.1 |
| 38 | H | 79.0 | A | 18.3 | PG | 2.7 | 20.0 |
| 39 | H | 79.0 | A | 17.8 | 1,3 XB | 3.2 | 20.0 |
| 40 | H | 69.3 | B | 27.9 | PG | 2.8 | 16.8 |
| 41 | H | 79.9 | B | 16.9 | PG | 3.2 | 20.1 |
| 42 | H | 67.7 | B | 29.6 | 1,3 XB | 2.7 | 16.8 |
| 43 | H | 78.0 | B | 18.9 | 1,3 XB | 3.1 | 19.9 |
| 44 | C | 100.0 | A | 87.0 | PG | 1.0 | 13.1 |

In the following Tables 3-5, the liquid isocyanates are formulated and evaluated. As would be realized from the Tables, one can increase flex modulus by increasing the allophanate content, at various chain extender levels. The Tables can be read using the following glossary of terms:

T-5000　　　　　a polyoxyalkylene polyamine from glycerine and propylene oxide having a molecular weight of about 5000, available from Texaco Chemical Co.

D-2000　　　　　a polyoxyalkylene polyamine having a molecular weight of 2000, available from Texaco Chemical Co.

DETDA (E-505)-　　an 80:20 mixture of 1-methyl-3,5-diethyl-2,4- and 2,6-phenylene diamine.

Zn Stearate -　　zinc stearate mold release agent.

| | | |
|---|---|---|
| L-5304 - | a silicone surfactant available from Union Carbide. | |
| M-3600 | a polyoxypropylene glycol having a molecular weight of about 2000. | |

TABLE #3

| POLYOL | 1 | 2 | 3 | 4 | 5 |
|---|---|---|---|---|---|
| COMPONENT (OH#) | | | | | |
| T-5000    34 | 53.49 | 53.49 | 53.49 | 53.49 | 53.49 |
| D-2000    56 | 22.93 | 22.93 | 22.93 | 22.93 | 22.93 |
| DETDA    629 | 22.00 | 22.00 | 22.00 | 22.00 | 22.00 |
| Zn Stearate 0 | 1.08 | 1.08 | 1.08 | 1.08 | 1.08 |
| L-5304    0 | .50 | .50 | .50 | .50 | .50 |
| **ISOCYANATE** | | | | | |
| NCO % | 17.50 | 17.60 | 17.60 | 17.50 | 17.60 |
| INDEX | 105 | 105 | 105 | 105 | 105 |
| Allophanate NCO % | 30.7 | 28.7 | 26.7 | 24.7 | 22.7 |
| Wt. % MDI (100/2) | 61.4 | 64.4 | 67.9 | 71.6 | 74.6 |
| Wt. % n-Butanol | 1.2 | 2.2 | 3.3 | 4.4 | 6.0 |
| Wt. % M3600 | 37.3 | 33.4 | 28.8 | 23.8 | 19.4 |

TABLE #3 - Continued

| PHYSICALS | 1 | 2 | 3 | 4 | 5 |
|---|---|---|---|---|---|
| Flex Modulus  psi | 30460 | 32044 | 5375 | 49725 | 68390 |
| Tensile     psi | 3454 | 3476 | 3690 | 3594 | 3742 |
| Elongation    % | 224.2 | 228.2 | 236.5 | 214.8 | 222.7 |
| Notched Izod ft.lb/in. | 9.20 | 8.10 | 9.94 | 11.19 | 10.70 |
| DIE "C" Tear  pli | 457.3 | 482.0 | 489.2 | 560.0 | 560.0 |

| PROCESSING | | | | | |
|---|---|---|---|---|---|
| MOLD TEMP  °C | 65 | 65 | 65 | 65 | 65 |
| POLYOL TEMP  °C | 45 | 45 | 45 | 45 | 45 |
| ISO TEMP  °C | 45 | 45 | 45 | 45 | 45 |
| DEMOLD TIME seconds | 30 | 30 | 30 | 30 | 30 |

| DEMOLD | | | | | |
|---|---|---|---|---|---|
| DEM. GRN STR | EXC | EXC | EXC | EXC | EXC |
| TEAR  (pli) | EXC | EXC | EXC | EXC | EXC |
| PROCESSING | EXC | EXC | EXC | EXC | EXC |

EP 0 641 812 A1

TABLE #4

| POLYOL | 6 | 7 | 8 | 9 | 10 |
|---|---|---|---|---|---|
| COMPONENT (OH#) | | | | | |
| T-5000    34 | 52.09 | 52.09 | 52.09 | 52.09 | 52.09 |
| D-2000    56 | 22.33 | 22.33 | 22.33 | 22.33 | 22.33 |
| DETDA    629 | 24.00 | 24.00 | 24.00 | 24.00 | 24.00 |
| Zn Stearate 0 | 1.08 | 1.08 | 1.08 | 1.08 | 1.08 |
| L-5304    0 | .50 | .50 | .50 | .50 | .50 |
| | | | | | |
| ISOCYANATE | | | | | |
| NCO % | 17.50 | 17.60 | 17.6 | 17.50 | 17.60 |
| INDEX | 105 | 105 | 105 | 105 | 105 |
| Allophanate NCO % | 30.7 | 28.7 | 26.7 | 24.7 | 22.7 |
| Wt. % MDI (100/2) | 61.4 | 64.4 | 67.9 | 71.6 | 74.6 |
| Wt. % n-Butanol | 1.2 | 2.2 | 3.3 | 4.4 | 6.0 |
| Wt. % M3600 | 37.3 | 33.4 | 28.8 | 23.8 | 19.4 |

EP 0 641 812 A1

TABLE #4 - Continued

| PHYSICALS | 6 | 7 | 8 | 9 | 10 |
|---|---|---|---|---|---|
| Flex Modulus psi | 40298 | 45310 | 51199 | 54515 | 78430 |
| Tensile psi | 3786 | 4061 | 3921 | 3829 | 3785 |
| Elongation % | 239.4 | 236.5 | 223.6 | 242.5 | 190.2 |
| Notched Izod ft.lb/in. | 8.90 | 8.10 | 9.94 | 11.19 | 10.70 |
| DIE "C" Tear pli | 544.5 | 587.1 | 594.5 | 545.7 | 588.3 |

| PROCESSING | | | | | |
|---|---|---|---|---|---|
| MOLD TEMP °C | 65 | 65 | 65 | 65 | 65 |
| POLYOL TEMP °C | 45 | 45 | 45 | 45 | 45 |
| ISO TEMP °C | 45 | 45 | 45 | 45 | 45 |
| DEMOLD TIME seconds | 30 | 30 | 30 | 30 | 30 |

| DEMOLD | | | | | |
|---|---|---|---|---|---|
| DEM. GRN STR | EXC | EXC | EXC | EXC | EXC |
| TEAR (pli) | EXC | EXC | EXC | EXC | EXC |
| PROCESSING | EXC | EXC | EXC | EXC | EXC |

EP 0 641 812 A1

TABLE #5

| POLYOL | | 11 | 12 | 13 | 14 | 15 |
|---|---|---|---|---|---|---|
| COMPONENT (OH#) | | | | | | |
| T-5000 | 34 | 50.69 | 50.69 | 50.69 | 50.29 | 52.29 |
| D-2000 | 56 | 21.73 | 21.73 | 21.73 | 21.73 | 21.73 |
| DETDA | 629 | 26.00 | 26.00 | 26.00 | 26.00 | 26.00 |
| Zn Stearate | 0 | 1.08 | 1.08 | 1.08 | 1.08 | 1.08 |
| L-5304 | 0 | .50 | .50 | .50 | .50 | .50 |
| | | | | | | |
| ISOCYANATE | | | | | | |
| NCO % | | 17.50 | 17.60 | 17.60 | 17.50 | 17.60 |
| INDEX | | 105 | 105 | 105 | 105 | 105 |
| Allophanate NCO % | | 30.7 | 28.7 | 26.7 | 24.7 | 22.7 |
| Wt. % MDI (100/2) | | 61.4 | 64.4 | 67.9 | 71.6 | 74.6 |
| Wt. % n-Butanol | | 1.2 | 2.2 | 3.3 | 4.4 | 6.0 |
| Wt. % M3600 | | 37.3 | 33.4 | 28.8 | 23.8 | 19.4 |

TABLE #5 - Continued

| | 11 | 12 | 13 | 14 | 15 |
|---|---|---|---|---|---|
| **PHYSICALS** | | | | | |
| Flex Modulus psi | 49537 | 52017 | 60002 | 70800 | 88870 |
| Tensile psi | 4499 | 4200 | 4364 | 4326 | 4033 |
| Elongation % | 251.9 | 228.6 | 234.1 | 236.0 | 198.2 |
| Notched Izod ft.lb/in. | 10.67 | 12.39 | 11.22 | 10.49 | 8.94 |
| DIE "C" Tear pli | 621.8 | 623.7 | 634.5 | 645.8 | 663.5 |
| **PROCESSING** | | | | | |
| MOLD TEMP °C | 65 | 65 | 65 | 65 | 65 |
| POLYOL TEMP °C | 45 | 45 | 45 | 45 | 45 |
| ISO TEMP °C | 45 | 45 | 45 | 45 | 45 |
| DEMOLD TIME seconds | 30 | 30 | 30 | 30 | 30 |
| **DEMOLD** | | | | | |
| DEM. GRN STR | EXC | EXC | EXC | EXC | EXC |
| TEAR (pli) | EXC | EXC | EXC | EXC | EXC |
| PROCESSING | EXC | EXC | EXC | EXC | EXC |

Although the invention has been described in detail in the foregoing for the purpose of illustration, it is to be understood that such detail is solely for that purpose and that variations constituting other embodiments can be made therein by those skilled in the art without departing from the spirit and scope of the invention except as it may be limited by the claims.

**Claims**

1. A stable, liquid MDI prepolymer containing an allophanate-modified MDI, having an NCO content of 12 to 32.5% comprising a reaction product of an aliphatic alcohol and diphenylmethane diisocyanate comprising about 2 to 60% by weight 2,4'-diphenylmethane diisocyanate and less than 6% by weight of the 2,2'-diphenylmethane diisocyanate, and the rest being 4,4'-diphenylmethane diisocyanate.

2. A stable, liquid MDI prepolymer containing an allophanate-modified MDI, having an isocyanate content of about 5 to 30%, comprising a reaction product of (A) the allophanate-modified MDI of Claim 1 and (B)(i) a high molecular weight organic material containing two or more active hydrogen groups which are hydroxyl groups, primary amine groups, secondary amine groups or a combination thereof, (ii) a low molecular weight diol, or (iii) a combination of (i) and (ii).

3. A process for preparing a stable, liquid MDI prepolymer containing an allophanate-modified MDI having an NCO content of about 12 to 32.5% comprising reacting an aliphatic alcohol and diphenylmethane diisocyanate comprising from 2 to 60% by weight 2,4'-diphenylmethane diisocyanate and less than 6% by weight of the 2,2'-diphenylmethane diisocyanate, and the rest being 4,4'-diphenylmethane

22

EP 0 641 812 A1

diisocyanate.

4. A process for preparing a liquid MDI prepolymer containing an allophanate-modified MDI having an NCO content of about 5 to 30% comprising reacting (A) an allophanate-modified MDI having an isocyanate content of about 12 to 32.5% which is a reaction product of (i) diphenylmethane diisocyanate comprising 4,4'-diphenylmethane diisocyanate containing from 2 to 60% by weight 2,4'-MDI and less than 6% by weight of the 2,2'-MDI, and (ii) an aliphatic alcohol, with (B) (i) a high molecular weight organic material containing two or more active hydrogen groups which are hydroxyl groups, primary amine groups, secondary amine groups or a combination thereof, (ii) a low molecular weight diol or (iii) a combination of (i) and (ii).

5. The process of Claim 3 wherein the allophanate-modified MDI is prepared at 60-110°C in the presence of a zinc acetylacetonate catalyst.

6. The process of Claim 5 wherein benzoyl chloride is a stopper for the catalyst.

7. A process for injecting a reaction mixture into a closed mold via a reaction injection molding (RIM) machine with the ratio of the components being such that the isocyanate index is about 80 to 120, said reaction mixture comprising: (i) an isocyanate reactive material, (ii) a chain extender, and (iii) a stable, liquid MDI prepolymer containing an allophanate-modified MDI having an isocyanate content of about 12 to 32.5% which is a reaction product of an aliphatic alcohol and an isomer composition of diphenylmethane diisocyanate comprising from 2 to 60% by weight 2,4'-diphenylmethane diisocyanate and less than 6% by weight of the 2,2'-diphenylmethane diisocyanate, and the rest being 4,4'-diphenylmethane diisocyanate.

8. The process of Claim 7 wherein the stable liquid, MDI prepolymer contains an allophanate-modified MDI having an NCO content of about 5 to 30% comprising a reaction product of (A) the allophanate-modified MDI, and (B) (i) an organic material containing two or more active hydrogen groups which are hydroxyl groups, primary amine groups, secondary amine groups or a combination thereof (ii) a low molecular weight diol, or (iii) a combination of (i) and (ii).

9. A molded article of matter which is prepared by the process of Claim 7.

10. A molded article of matter which is prepared by the process of Claim 8.

23

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 031 650 (I.C.I.)<br>* claims 1-6 *<br>* page 3, line 12 - line 24 *<br>* page 5, line 10 - page 6, line 29 *<br>* examples 1,5 *<br>--- | 1,3 | C08G18/78<br>C08G18/10<br>C08G18/12<br>C07C273/18 |
| X | EP-A-0 393 903 (I.C.I.)<br>* claims 1,3,6,7,9,11 *<br>* examples 1,3 *<br>--- | 1 | |
| D,A | US-A-4 738 991 (N. THIRUMURTI)<br>* claims 1,3-6 *<br>* column 1, line 49 - column 2, line 33 *<br>* column 2, line 46 - column 3, line 9 *<br>--- | 1 | |
| A | US-A-4 539 157 (J.E. DEWHURST ET AL.)<br>* claims 1-11 *<br>* column 2, line 47 - line 68 *<br>* column 3, line 33 - line 38 *<br>--- | 1 | |
| A | FR-A-2 380 309 (MOBAY)<br>* claims 1-3 *<br>* page 2, line 12 - page 3, line 35 *<br>----- | 1 | TECHNICAL FIELDS SEARCHED (Int.Cl.6)<br><br>C08G<br>C07C |

The present search report has been drawn up for all claims

| Place of search | Date of completion of the search | Examiner |
|---|---|---|
| THE HAGUE | 14 December 1994 | Van Puymbroeck, M |

EPO FORM 1503 03.82 (P04C01)